# EUROPÄISCHE PATENTSCHRIFT

(11) **EP 2 614 060 B1**
(45) Veröffentlichungstag und Bekanntmachung des Hinweises auf die Patenterteilung: **31.08.2016**
(21) Anmeldenummer: 11755294.3
(22) Anmeldetag: 30.08.2011
(51) Int. Cl.: C07D 417/06, C07D 417/14, A61K 31/5415, A61P 25/28, G01N 33/68

(54) **THIETHYLPERAZIN ZUR BEHANDLUNG EINER BETA-AMYLOIDOPATHIE ODER ALPHA-SYNUCLEOPATHIE**
THIETHYLPERAZINE FOR TREATING A BETA-AMYLOIDOPATHY OR AN ALPHA-SYNUCLEOPATHY
THIÉTHYLPÉRAZINE POUR LE TRAITEMENT D'UNE PATHOLOGIE BÉTA-AMYLOÏDE OU D'UNE ALPHA- SYNUCLÉOPATHIE

(30) Priorität: 07.09.2010 DE 102010044561
(43) Veröffentlichungstag der Anmeldung: 17.07.2013
(60) Teilanmeldung: 13190419.5
(73) Patentinhaber: Immungenetics AG, 18055 Rostock (DE)
(72) Erfinder: PAHNKE, Jens, Prof., 39104 Magdeburg (DE)
(74) Vertreter: Herzog, Fiesser & Partner Patentanwälte PartG mbB
(86) Internationale Anmeldenummer: PCT/EP2011/064893
(87) Internationale Veröffentlichungsnummer: WO 2012/031941

(56) Entgegenhaltungen:
- WO-A1-96/04915
- WO-A1-2008/092898
- WO-A2-2008/133884
- CIRRITO J R ET AL: "P-glycoprotein deficiency at the blood-brain barrier increases amyloid-beta deposition in an Alzheimer disease mouse model", JOURNAL OF CLINICAL INVESTIGATION, AMERICAN SOCIETY FOR CLINICAL INVESTIGATION, US, Bd. 115, Nr. 11, 1. November 2005 (2005-11-01), Seiten 3285-3290, XP003015274, ISSN: 0021-9738, DOI: 10.1172/JCI25247
- PAHNKE JENS ET AL: "Clinico-pathologic function of cerebral ABC transporters - Implications for the pathogenesis of Alzheimer's disease", CURRENT ALZHEIMER RESEARCH, Bd. 5, Nr. 4, August 2008 (2008-08), Seiten 396-405, XP009160059, ISSN: 1567-2050
- DEELEY R G ET AL: "Substrate recognition and transport by multidrug resistance protein 1 (ABCC1)", FEBS LETTERS, ELSEVIER, AMSTERDAM, NL, Bd. 580, Nr. 4, 13. Februar 2006 (2006-02-13), Seiten 1103-1111, XP028030467, ISSN: 0014-5793, DOI: 10.1016/J.FEBSLET.2005.12.036 [gefunden am 2006-02-13] in der Anmeldung erwähnt
- Markus Krohn et al.: "Cerebral amyloid-[beta] proteostasis is regulated by the membrane transport protein ABCC1 in mice", The Journal of Clinical Investigation, Bd. 121, Nr. 10 1. September 2011 (2011-09-01), Seiten 3924-3931, XP55029425, ISSN: 1558-8238, DOI: 10.1172/JCI57867DS1) Gefunden im Internet: URL:http://static.jci.org/content_assets/m anuscripts/57000/57867/JCI57867.v2.pdf [gefunden am 2012-06-11]

## Beschreibung

Die Akkumulation von Proteinen oder Proteinfragmenten (Peptiden) im Gehirn ist ein signifikantes Merkmal von altersabhängigen neurodegenerativen Erkrankungen. Bei der Alzheimer Demenz (Alzheimer disease, AD) und der zerebralen β-Amyloidangiopathie (CAA) ist die Aggregation vonβ-Amyloid-Peptiden(Aβ) krankheitsauslösend, wobei der zugrunde liegende Mechanismus nicht bekannt ist. Die Aß-Proteostase, d.h. das Gleichgewicht von Produktion und Abbau/Abtransport mittels Rezeptoren oder Proteasen, ist bei der AD und CAA gestört. Der Entfernung der Aß-Peptide durch zelluläre Transporter (ABC Transporter) wurde jedoch bisher wenig Beachtung beigemessen. Bei der Parkinson-Krankheit (Morbus Parkinson) akkumuliert das Protein α-Synuclein, welches unter anderem die Dopamin-Ausschüttung in der Substantia nigra reguliert. Bei der α-Synucleinopathie Morbus Parkinson ist es bekannt, dass ABC-Transporter eine entscheidende Rolle für den Transport spielen (Kortekaas et al., Ann Neurol 2005, 57, 176-179). Hierbei existieren mehrere Subfamilien A-G, die wechselseitig verschiedene Substrate transportieren können (Metaboliten, Medikamente, Peptide, Proteine, Ionen) und sogar in der Lage sind sich gegenseitig in der Transportfunktion zu ersetzen (z.B. ABCB1 und ABCC1, Tao et al. Cancer Chemotherapy and Pharmacology, 64, 5, 961-969).

WO2008/092898A1 beschreibt eine Kombination aus zwei Wirkstoffen zur Behandlung von Krankheiten, die durch Proteinaggregation hervorgerufen werden. Bei einem der Wirkstoffe handelt es sich um ein Phenothiazin-Derivat, wobei Thiethylperazin nicht umfasst wird. WO96/04915A1 beschreibt die Behandlung der Alzheimer-Krankheit mittels Phenothiazin-Derivaten oder Thioxanthenen, wobei Thiethylperazin nicht umfasst wird. WO2008/133884A2 beschreibt die Behandlung einer Vielzahl neurodegenerativer Erkrankungen mit einer Vielzahl von Wirkstoffen. Bei einem der Wirkstoffe handelt es sich um Thiethylperazin. Alle experimentellen Daten beziehen sich jedoch auf die Huntington-Krankheit.

Mittels verschiedener gentechnisch veränderter Mausmodelle konnte gezeigt werden, dass der ABC-Transporter (gemeinsames Strukturelement der ABC-Transporter ist eine ATPbindende Kassette und eine Transportpore) ABCC1, ein wichtiger Protein/Peptid-Transporter, insbesondere Aß-Transporter ist, der außergewöhnliche funktionelle Auswirkungen auf die zerebrale Protein-Akkumulation hat. ABCC1 ist ebenso ein wichtiger α-Synuclein-Transporter.

Nachfolgend werden die Untersuchungen zur Transporter-Aktivität exemplarisch am Aβ-Transport dargestellt.

Zur Bestimmung der ABCC1-Aktivität *in vivo* wurde bei APP-exprimierenden, transgenen Mäusen jeweils der ABCB1-, der ABCG2- oder der ABCC1-Transporter gentechnisch entfernt (knockout Mäuse).

Dabei wurde gefunden, dass:
i) die Menge von Aβ in den Mäusen, denen der ABCC1
   Transporter fehlte, um das 12-fache gesteigert war,
ii) der ABCB1-Transporterverlust nur zu einer 3-fachen Erhöhung führt und
iii) der ABCG2-Verlust keine Aβ-akkumulierende Auswirkung hat.

Aufgabe der vorliegenden Erfindung war es daher, Substanzen bereit zu stellen, welche den ABCC1-Transporter in geeigneter Weise beeinflussen, um so neurodegenerative Erkrankungen, insbesondere β-Amyloidopathien oder α-Synucleopathien, behandeln zu können. Diese Aufgabe wurde mit dem 2-(R²-Thio)-10-[3-(4-R¹-piperazin-1-yl)propyl]-10*H*-phenothiazin gemäß Anspruch 1 gelöst. Weitere bevorzugte Ausführungsformen ergeben sich aus den abhängigen Ansprüchen.

In anderen Worten wurde die Aufgabe durch das 2-(R²-Thio)-10-[3-(4-R¹-piperazin-1-yl)propyl]-10H-phenothiazin gemäß der allgemeinen Formel I 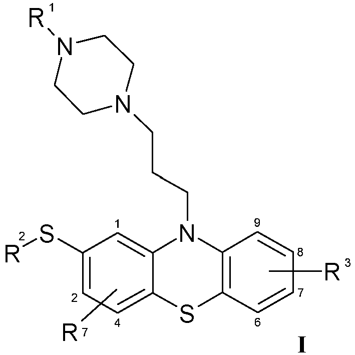 wobei der Rest R eine Methylgruppe, der Rest R² eine Ethylgruppe und die Reste R³ und R⁷ Wasserstoff sind, zur Behandlung einer β-Amyloidopathie oder einer α-Synucleinopathie, welche mit einer zerebralen Proteinablagerung und einer verminderten Aktivität des zerebralen ABCC1-Transporters einhergehen, gelöst.

Weiterhin besteht sowohl bei α-Synucleinopathien als auch bei β-Amyloidopathien ein Bedarf nach Möglichkeiten, diese Krankheiten zu erkennen bzw. zu diagnostizieren oder prädiagnostizieren.

Aufgabe der Spezifikation war auch die Bereitstellung eines Verfahrens, mit welchem α-Synucleinopathien als auch β-Amyloidopathien diagnostiziert oder prädiagnostiziert werden können. Diese Aufgabe wird durch ein Verfahren zur Diagnose oder Prädiagnose einer β-Amyloidopathie oder α-Synucleopathie oder zur Ermittlung des Risikos eines Probanden, an einer solchen Krankheit zu erkranken, gelöst, wobei der Proband bereits Substanzen zu sich nimmt, welche über den zerebralen ABCC1-Transporter transportiert werden, bestehend aus den folgenden Schritten:
a) Bestimmung der Menge der eingenommenen Substanz in Körperflüssigkeitsproben des Probanden zu einem bestimmten Zeitpunkt;
b) Wiederholung der Bestimmung des Schrittes a) zu mindestens einem weiteren, späteren Zeitpunkt;
c) Vergleich der in Schritt a) und b) ermittelten Mengen mit Mengen, welche zu gleichen Zeitpunkten als charakteristisch für Probanden definiert worden waren, die zur Zeit der Probennahme keine klinischen Anzeichen einer β-Amyloidopathie oder α-Synucleopathie zeigten.

Dass der Proband bereits mindestens einen Stoff, welcher über den zerebralen ABCC1-Transporter transportiert wird, zu sich nimmt, bedeutet, dass dieser Stoff nicht erst verabreicht werden muss. Vielmehr ist er im Körper des Probanden bereits vorhanden, beispielsweise aufgrund einer medikamentösen Behandlung einer anderen Erkrankung. Die Körperflüssigkeitsproben des Probanden, welche untersucht werden, sind vorzugsweise Proben aus Blutplasma, Blutserum und/oder Nervenwasser.

Die β-Amyloidopathie ist vorzugsweise eine Alzheimer Demenz, die α-Synucleinopathie vorzugsweise die Parkinson'sche Erkrankung. Gegebenenfalls kann die α-Synucleinopathie auch eine Lewy-Körperchen-Demenz (DLB) sein. Substanzen, welche über den zerebralen ABCC1-Transporter transportiert werden, sind vorzugsweise ausgewählt aus Antibiotika (z.B. Difloxacin, Grepafloxacin), Virostatika/antiviralen Medikamenten (z.B. Saquinavir, Ritonavir), Antiallergika/ Antihistaminika (z.B. Cimetidin), Herz-Kreislauf-Medikamenten (z.B. Verapamil), Antidepressiva (z.B. Citalopram), Antihyperurikämika (z.B. Probenecid), Zytostatika (z.B. Methotrexat, Etoposit, Edatrexat, ZD1694), Vitaminen/Vitaminanaloga (z.B. Methotrexat, Folsäure, L-Leucovorin), Antiphlogistika (z.B. Indomethacin), Antiepileptika (z.B. Valproinsäure), Hormonen/Hormonderivaten (z.B. 17ß-Estradiol), Leukotrienen (z.B. LTC4), fluoreszierenden Proben (z.B. Calcein, Fluo-3, BCECF, SNARF), GSH-, Sulfat- oder Glucuronidgekoppelten Metaboliten natürlicher Stoffe (endogen produziert), Toxinen oder von Medikamenten (z.B. 2,4-Dinitrophenyl-SG, Bimane-SG, N-Ethylmaleimid-SG, Doxorubicin-SG, Thiotepa-SG, Cyclophosphamid-SG, Melphalan-SG, Chlorambucil-SG, Ethacrynsäure-SG, Metolachlor-SG, Atrazin-SG, Sulforaphan-SG, Aflatoxin B1-Epoxid-SG, 4-Nitroquinolin 1-oxid-SG, As(SG)3, Etoposid-Gluc, 4-(Methylnitrosamino)-1-(3-pyridyl)-1-butanol (NNAL)-3β-O-Gluc, SN-38-gluc, 4-Methylumbelliferyl-β-d-gluc, 6-Hydroxy-5,7-dimethyl-2-methylamino-4-(3-pyridylmethyl)-benzothiazolsulfat (E3040S)-Gluc, Leukotrien C4, Prostaglandin A2-SG, 15-deoxy-Δ12,14 prostaglandin J2-SG, Hydroxynonenal-SG , 17β-Estradiol-17-β-d-gluc, Glucuronosylbilirubin, Bis-lucuronosylbilirubin, Hyodeoxycholate-6-α-Gluc, Estron-3-Sulfat, Dehydroepiandrosteron-Sulfat, Sulfatolithocholat) (siehe auch Deeley RG et al.: Substrate recognition and transport by multi drug resistance protein 1 (ABCC1), FEBS letters 2006, 580 (4), pp. 1103-1111.)

Diese indirekte Analyse der Transporteraktivität des ABCC1-Transporters kann zur Diagnose/Prädiagnose einer entsprechenden Krankheit genutzt werden. Bei Probanden, welche bereits auf anderen Wegen ABCC1-transportable Substanzen zu sich nehmen, kann das Profil der Wirkstoffkonzentration in Körperflüssigkeiten, vorzugsweise Blutplasma, -serum und/oder Nervenwasser, untersucht werden. Eine zeitabhängige Messung zeigt für Probanden, bei welchen eine verminderte ABCC1-Transporteraktivität gegenüber gesunden Probanden vorliegt, eine verzögerte bzw. verschobene Substanz-Konzentrationskurve (Konzentration c über Zeit t aufgetragen), d.h. das Maximum der Kurve tritt zeitlich verändert auf.

Wenn sich eine verschobene Kurve gegenüber dem gesunden Fall ergibt, ist dies ein Anzeichen für eine veränderte ABCC1-Transportaktivität. Dies bedeutet, dass dann auch Substanzen wie Aβ oder α-Synuclein schlechter transportiert werden und ist somit ein Anzeichen für eine entsprechende Erkrankung.

Sowohl die Mausmodelle als auch die pharmakologische Beeinflussung des ABCC1 zeigen, dass dieser ein wichtiger zellulärer Transmembrantransporter für das Aß-Protein ist und implizieren, dass die Blut-Hirn-Schranke und der Plexus choroideus eine Schlüsselposition für die Aß-Ausscheidung aus dem Gehirn einnehmen. Es konnte gezeigt werden, dass die selektive pharmakologische Aktivierung des ABCC1-Transporters die zerebrale Belastung mit Aβ signifikant verringert und so therapeutisch zur Behandlung von Erkrankungen mit gestörter Hirn-Proteostase verwendbar ist. Weiterhin kann die Analyse der Transporteraktivität des ABCC1-Transporters wie oben beschrieben zur indirekten oder direkten Diagnose/Prädiagnose einer entsprechenden Krankheit genutzt werden. Die direkte Analyse wäre über die Verabreichung von Substanzen, welche über den ABCC1-Transporter transportiert werden, und deren Bestimmung, möglich. Die indirekte Analyse wurde bereits weiter oben erläutert.

Veränderungen von Exportmechanismen, welche in Zusammenhang mit ABC-Transportern stehen, können das temporale Aggregationsprofil von Aβ und anderen Hirnproteinen substantiell beeinflussen. Demzufolge wirkt sich eine Beeinflussung der Funktion des ABCC1-Transporters auf das Risiko an neurodegenerativen Erkrankungen, insbesondere an Alzheimer, zu erkranken positiv aus. "Behandlung neurodegenerativer Erkrankungen" umfasst in diesem Sinne die Prophylaxe als auch die Behandlung bereits bestehender Erkrankungen.

Die Rolle der ABC-Transporter bei der Aß-Ausscheidung wurde zuerst derart untersucht, dass nachgewiesen wurde, dass ABCC1 in der Lage ist, Aβ zu transportieren. Hierzu wurden *in vitro* Transwell-Assays mit Endothelzellen (endothelialcelltranswellassay, ECTA) von primären, kultivierten, Kapillarendothelzellen aus Mausgehirnen verwendet (Zellkulturansatz):

Primäre Kulturen von Endothelzellen aus Gehirnkapillaren von ABCB1-defizienten, ABCC1-defizienten (knock out) Mäusen und von Kontrollmäusen (C57BI/6, FVB/N) wurden genutzt, um die Aß-spezifische Transportaktivität zu untersuchen. Der Transport von Aβ aus dem abluminalen (Gehirn) in das luminale(Blut)-Kompartment ist bei ABCB1-defizienten und ABCC1-defizienten Endothelien beeinträchtigt. Die mittlere Aß-Transportrate während der ersten sechs Stunden nach Gabe von Aß-Peptiden (Aβ42) betrug 2,2pg/min für die Kontrollzellen. Im Gegensatz dazu erreichten die ABCC1-defizienten Zellen nur die halbe Transportkapazität (1,0pg/min). In den ABCB1-defizienten Zellen war der Aß-Transport nahezu nicht vorhanden (0,3pg/min). Weitere Untersuchungen an Kapillarendothelien und Zellen aus dem Plexus choroideus ergaben, dass der Transporter ABCB1 stark in Gehirnkapillarendothelien exprimiert wird, wohingegen die endotheliale ABCC1-Expression in Hirnkapillaren geringer ist.

Anhand neu generierter ABC-Transporter-defizienter Alzheimer Mausmodelle wurde dann die relative Signifikanz von Mitgliedern der ABC-Transporterfamilie *in vivo* untersucht. Die gentechnisch veränderten Mäuse weisen jeweils eine Defizienz (knock out) an spezifischen ABC-Transportern ABCG2, ABCB1 oder ABCC1auf.

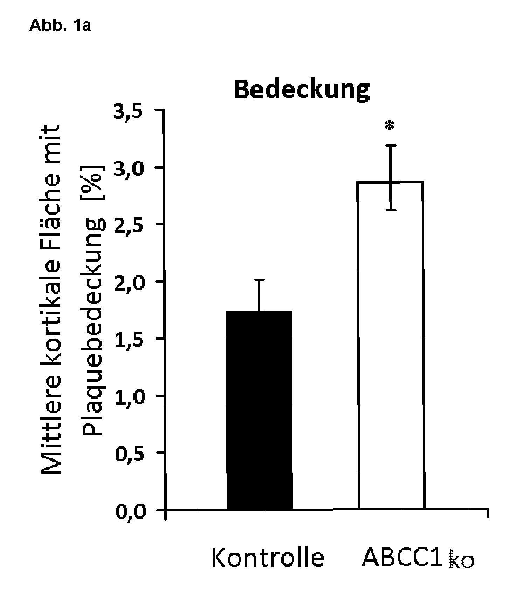

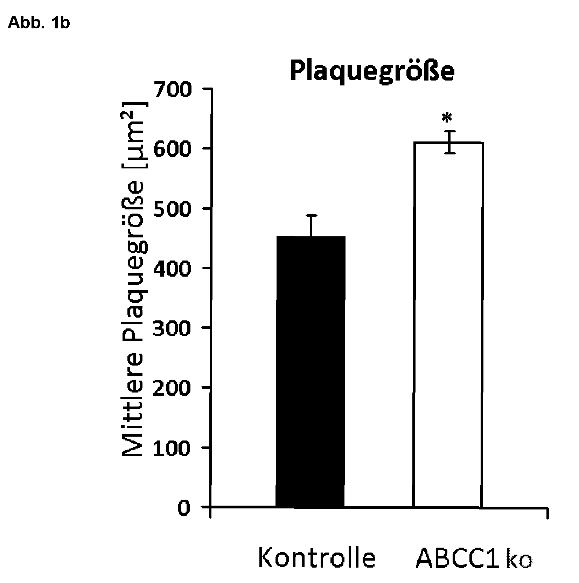

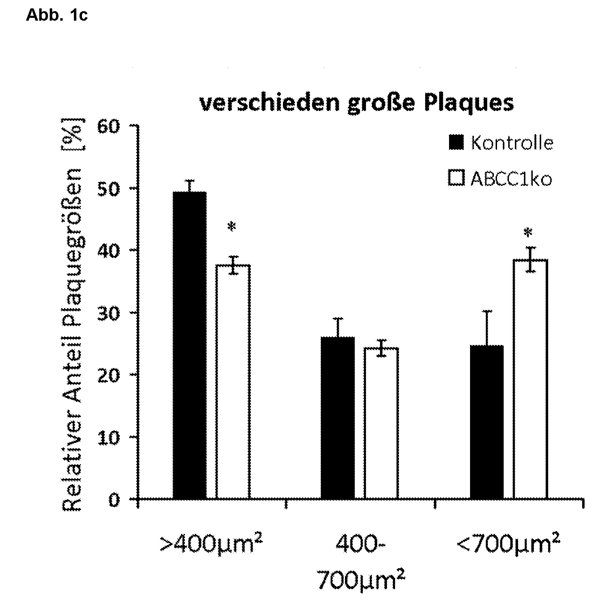

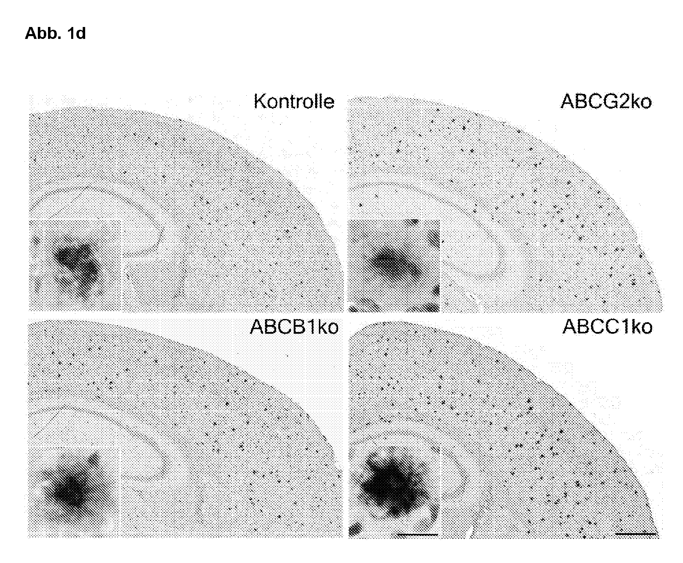

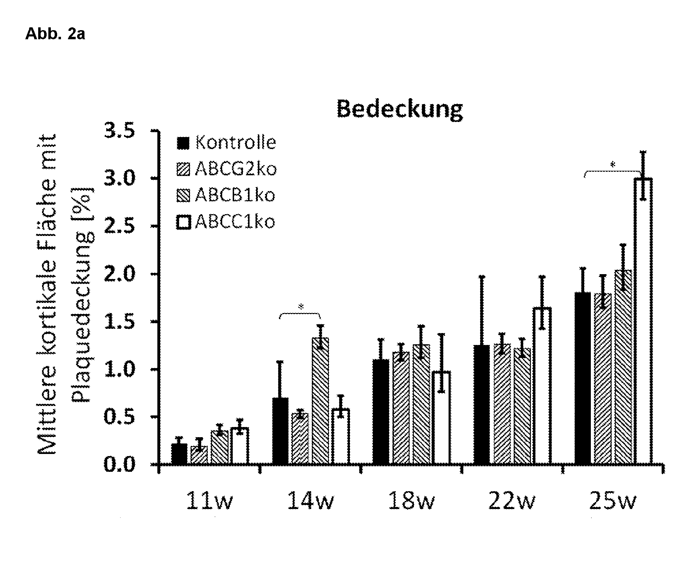

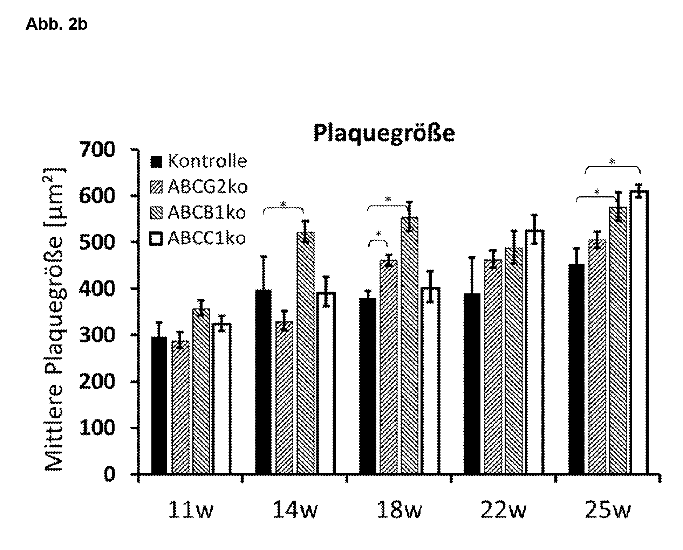

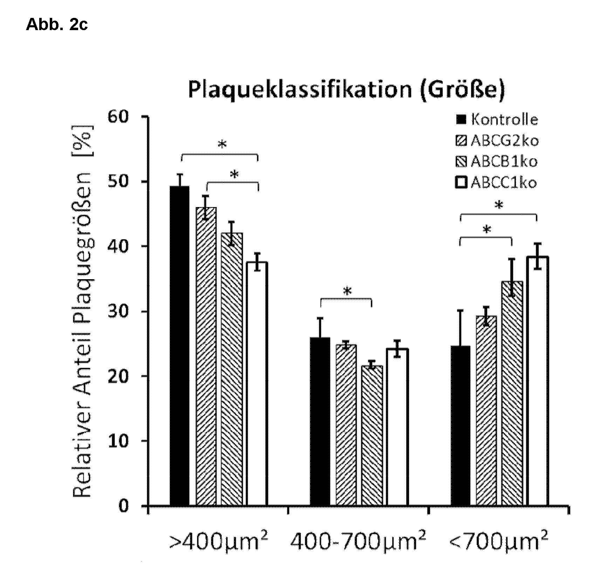

Die Aß-Immunohistochemie von Gehirnschnitten zeigte:
i) signifikante Anstiege in der kortikalen Anzahl und der Größe Aß-positiver Plaques in ABCC1-defizienten Mäusen verglichen zu Kontrollmäusen (siehe Fig. 1 und 2a-c).
ii) ABCB1-defizienteMäuse zeigten einen geringeren Anstieg der Anzahl und Größe von Aβ-Plaques als ABCC1-defiziente Mäuse.
iii) Zwischen Kontrollmäusen und ABCG2-defizienten Mäusen konnte kein signifikanter Unterschied festgestellt werden (Fig. 2a-c).

Zur Bestimmung der Menge an pufferlöslichem Aβ (größtenteils Monomere und kleinere Oligomere) und an Guanidin-löslichem Aβ (größtenteils fibrilläres bzw. aggregiertes Material) wurden enzymgekoppelte Immunadsorptionstests (Enzyme-linkedimmunoabsorbentassays, ELISAs) für Aβ verwendet.

In Übereinstimmung mit den morphologischen Ergebnissen aus der Immunohistochemie zeigten die ABCC1-defizienten Mäuse einen signifikanten Anstieg bei aggregiertem Aβ im Vergleich zu den Kontrollmäusen zu allen Messungszeitpunkten. Die zerebrale Belastung mit Aβ war in einem Alter von 25 Wochen am stärksten. Zu diesem Zeitpunkt waren die Aß-Werte (Aβ42) 12-mal höher als bei den Kontrollmäusen. Pufferlösliches Aβ stieg mit dem Alter ebenfalls an, aber nach 25 Wochen, zum Zeitpunkt der höchsten Plaques-Belastung, fielen die Werte des löslichen Aßs in der ABCC1-defizienten Gruppe stark ab.

Weitere Untersuchungen wurden durchgeführt, welche weitere Belege für den Zusammenhang zwischen dem ggf. fehlenden Abtransport durch ABCC1 und der Aggregation von Aβ lieferten.

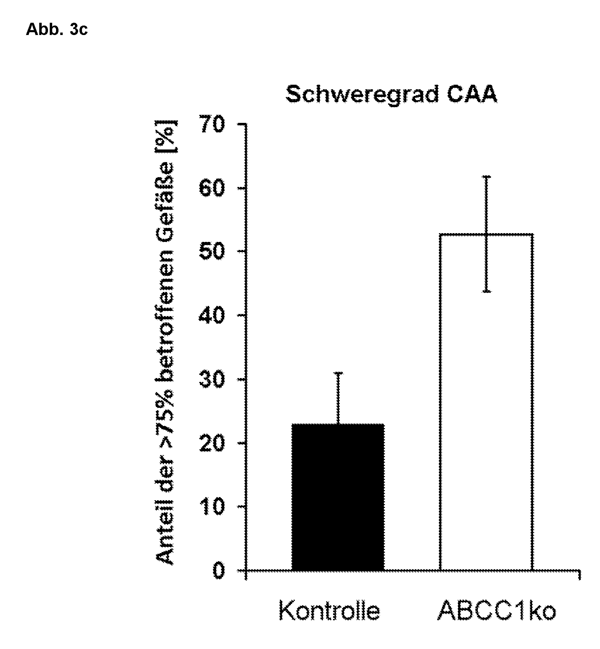

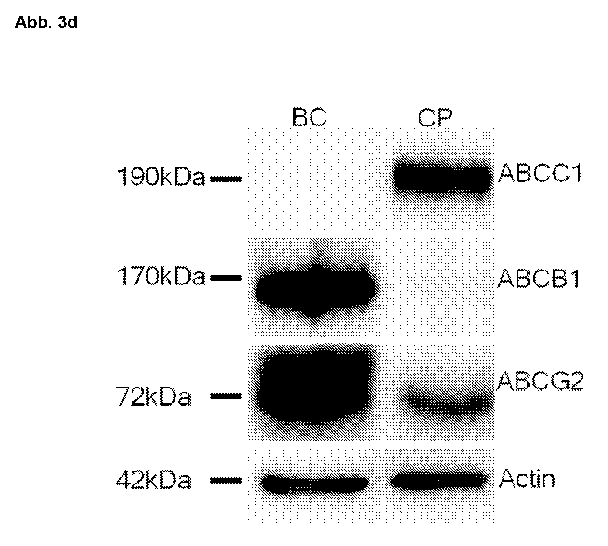

Die Transportkinetiken von ABC-Transportern hängen unter anderem von spezifischen Protein-/Peptidcharakteristika wie der spezifischen Ladung ab. Die Dutch-type-Variante des Amyloid-Precursor-Proteins (holländische Mutante, APP_{dt}), welche eine zusätzliche negative Ladung nahe der Schnittstelle der α-Sekretase des APP einführt und so zu einer schweren, zerebralen Amyloidangiopathie (CAA) führt, beeinflusst die Eliminierung des Aβ_{dt} über die Blut-Hirn-Schranke. Die Western-blot-Analysen von Gehirnkapillaren und Plexuschoroideus (CP) aus Kontrollmäusen zeigten eine starke Expression von ABCB1 in zerebralen Kapillar-endothelien (BC) und von ABCC1 im CP (Fig. 3d). Da ABC-Transporter eine wichtige Rolle bei der Eliminierung des Aβ spielen, wurde angenommen, dass ABC-Transporter-defiziente (an der Blut-Hirn-Schranke und an der Blut-Plexuschoroideus-Schranke) APP_{dt}-transgene Mäuse eine verstärkte Akkumulation von Aβ_{dt} in meningealen Gefäßen aufweisen. Der Grad der CAA in den ABC-defizienten APP_{dt}-Mäusen wurde im Alter von 24 Monaten quantifiziert. In Übereinstimmung mit der Annahme waren mindestens 51 % der Gefäße schwer beeinträchtigt (>75% der Gefäßwand mit Aβ beladen) bei den ABCC1-defizienten Tieren gegenüber 23% bei den Kontrollen (Fig. 3c).

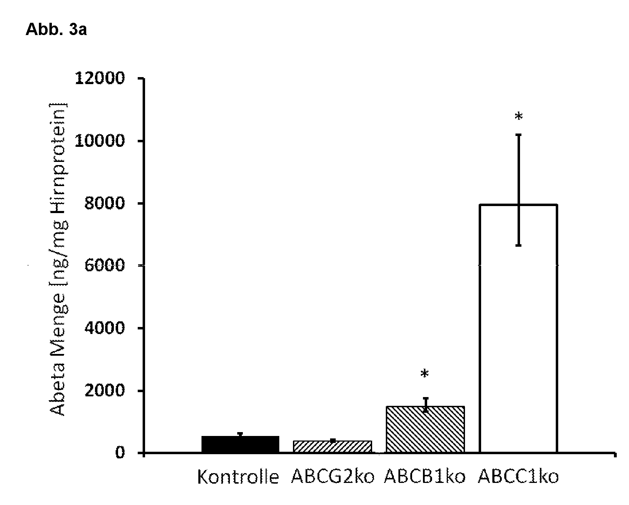

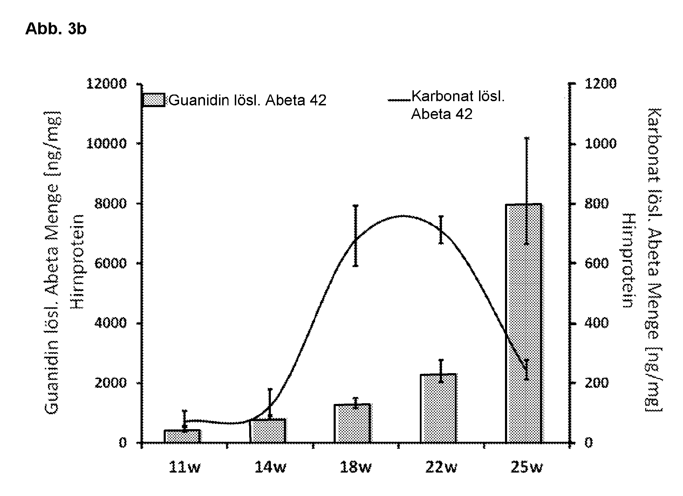

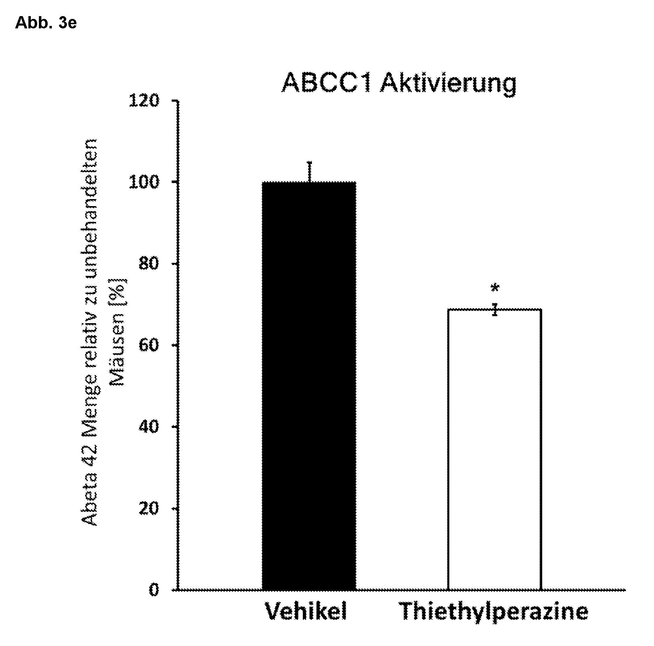

Auf Basis dieser Ergebnisse wurde untersucht, inwieweit der Gehalt an löslichem Aβ im Gehirn durch wirkstoffvermittelte Aktivierung von ABC-Transportern verringert/beeinflusst werden konnte. Mäuse mit Amyloidablagerungen wurden für 30 Tage mit dem anti-emetischen Thiethylperazin (Torecan^{®},2-(Ethylthio)-10-[3-(4-methylpiperazin-1-yl)propyl]-10*H-*phenothiazin) behandelt. Zweimal täglich wurden 3mg/kg Körpergewicht intramuskulär verabreicht. Die prophylaktische Behandlung begann bereits bevor die Mäuse senile Plaques ausbildeten. ELISA-Messungen der behandelten Tiere zeigten eine Reduktion der Aß-Menge von mindestens 31% bei den behandelten Mäusen im Vergleich zu mit Vehikel-behandelten Tieren (Vehikel = Wasser) (Fig. 3e). Die Ergebnisse sind graphisch in Fig. 3 wiedergegeben.

Die Fähigkeit zur Entfernung von Aβ erwies sich als ein Schlüsselfaktor bei der Regulation der intrazerebralen Akkumulation von Aβ.

Als besonders effizienter Aktivator des ABCC1-Transporters erwies sich Thiethylperazin (Torecan^{®}). Weitere Derivate ausgehend vom selben Grundgerüst zeigten ebenfalls gute Ergebnisse bei der Aktivierung des ABCC1-Transporters. Die entsprechenden Derivate sind in der allgemeinen Formel I dargestellt 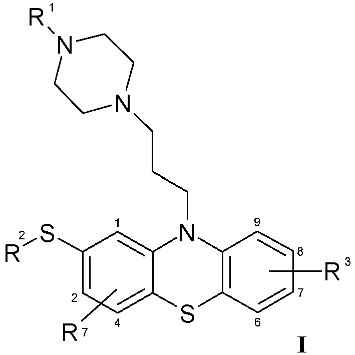 wobei die Reste
R¹ und R² gleich oder verschieden sind und jeweils unabhängig voneinander C₁-C₆-Alkylgruppen sind, welche unabhängig voneinander gegebenenfalls einen weiteren Substituenten ausgewählt aus Alkyl-, Aryl-, Acyl-(vorzugsweise Acetyl-), Amino-, Nitro-, Sulfonyl-, Hydroxyl-, Alkoxy-, Aryloxy-, Arylthio-, Alkylthio-Gruppe und Halogenatom aufweisen, wobei die jeweiligen Alkylgruppen gegebenenfalls mindestens ein weiteres Halogenatom aufweisen und der Rest
R³ sich an einer der Positionen 6-9 des Phenothiazin-Ringsystems, vorzugsweise an Position 6, 7 oder 8, befindet und ein Wasserstoffatom oder eine Alkyl-, Aryl-, Acyl-(vorzugsweise Acetyl-), Amino-, Nitro-, Sulfonyl-, Hydroxyl-, Alkoxy-, Aryloxy-, Arylthio-oder Alkylthio-Gruppe oder ein Halogenatomist, wobei die jeweiligen Alkylgruppen gegebenenfalls mindestens ein weiteres Halogenatom aufweisen,oder eine NR⁴R⁵-oder OR⁶-Gruppe ist, wobei R⁴, R⁵ und R⁶ gleich oder verschieden sind und jeweils unabhängig voneinander ausgewählt sind aus Wasserstoff und C₁-C₃-Alkylgruppe und der Rest
R⁷ sich an einer der Positionen 1, 2 oder 4 des Phenothiazin-Ringsystems, vorzugsweise an Position 2 oder 4, befindet und ein Wasserstoffatom oder eine Alkyl-, Aryl-, Acyl-(vorzugsweise Acetyl-), Amino-, Nitro-, Sulfonyl-, Hydroxyl-, Alkoxy-, Aryloxy-, Arylthio-oder Alkylthio-Gruppe oder ein Halogenatom ist, wobei die jeweiligen Alkylgruppen gegebenenfalls mindestens ein weiteres Halogenatom aufweisen, oder eine NR⁸R⁹-oder OR¹⁰-Gruppe ist, wobei R⁸, R⁹ und R¹⁰ gleich oder verschieden sind und jeweils unabhängig voneinander ausgewählt sind aus Wasserstoff und C₁-C₃-Alkylgruppe.

Diese Derivate sind entsprechend gut geeignet zur Behandlung neurodegenerativer Erkrankungen, insbesondere von β-Amyloidopathien oder α-Synucleinopathien, wobei Behandlung wie oben erwähnt sowohl die Prophylaxe als auch die Behandlung bereits bestehender Erkrankungen umfasst. Das Halogenatom/die Halogenatome sind vorzugsweise ausgewählt aus Fluor und Chlor. Die Acylgruppen (-(C=O)-R) der Reste R^{1, 2, 3, 7} sind vorzugsweise Acetylgruppen (-C(=O)CH₃). Vorzugsweise sind die Reste R¹ und R² gleich oder verschieden und jeweils unabhängig voneinander eine C₁-C₆-Alkylgruppe oder eine C₁-C₆-Alkylgruppe (bevorzugt C₁-Alkyl), substituiert mit einer Acetylgruppe, sowie die Reste R³ und R⁷ Wasserstoff oder eine Acetylgruppe. Bevorzugt sind die Reste R¹ und R² gleich oder verschieden und jeweils unabhängig voneinander eine C₁-C₃-Alkylgruppe. Weiterhin ist es bevorzugt, dass die Reste R³ und R⁷ Wasserstoff sind. Besonders bevorzugt ist es, wenn der Rest R¹ eine Methylgruppe, der Rest R² eine Ethylgruppe und die Reste R³ und R⁷ Wasserstoff sind (Thiethylperazin, Torecan^{®}). Bei der Verwendung zur Behandlung neurodegenerativer Erkrankungen hat es sich als vorteilhaft erwiesen, den 2-(R²-Thio)-10-[3-(4-R¹-piperazin-1-yl)propyl]-10*H*-phenothiazinen weitere Wirkstoffe, vorzugsweise 1-Benzhydrylpiperazine, höchst bevorzugt1-Benzhydryl-4-cinnamyl-piperazin (Cinnarizin), zuzusetzen.

Verschiedene neurodegenerative Erkrankungen können mit dem erfindungsgemäßen 2-(R²-Thio)-10-[3-(4-R¹-piperazin-1-yl)propyl]-10*H*-phenothiazin-Derivat behandelt werden oder sind mittels der oben beschriebenen indirekten Analyse diagnostizierbar. In einer besonders bevorzugten Ausführungsform ist die neurodegenerative Erkrankung eine β-Amyloidopathie, insbesondere Alzheimer Demenz (AD).Eine andere Ausführungsform betrifft den Fall, dass die neurodegenerative Erkrankung eine α-Synucleinopathie, insbesondere Parkinson'sche Erkrankung (PD) ist. Beide Erkrankungen, d.h. β-Amyloidopathie und α-Synucleinopathien, sind durch zerebrale Proteinablagerungen charakterisiert, welche anhand einer Aktivierung des ABCC1-Transporters behandelt oder anhand dessen Aktivität diagnostiziert werden können.

Andere, ebenfalls über Aktivierung des ABCC1-Transporters behandelbare oder anhand der ABCC1-Transporteraktivität diagnostizierbare Erkrankungen, sind nachfolgend genannt. So ist eine weitere, behandelbare Erkrankung die Lewy-Körperchen-Demenz (LBD). Diese ist ebenfalls durch eine zerebrale Proteinaggregation gekennzeichnet, d.h. ist eine α-Synucleinopathie wie Parkinson.

Eine andere nicht-erfindungsgemäße Ausführungsform betrifft den Fall, dass die neurodegenerative Erkrankung die Huntington'sche Erkrankung (HD) ist. Eine andere nicht-erfindungsgemäße Ausführungsform betrifft den Fall, dass die neurodegenerative Erkrankung eine Prion-Erkrankung, insbesondere Creutzfeld-Jacob-Erkrankung (CJD) oder Fatale Familiäre Insomnie (FFI), ist. Eine andere nicht-erfindungsgemäße Ausführungsform betrifft den Fall, dass die neurodegenerative Erkrankung eine Tauopathie, insbesondere Cortico-Basale-Degeneration (CBD), Steel-Richardson-Olszewski-Syndrom (PSP, progessivesupranuclearpalsy- progressive supranukleäre Blickparese) oder Pick'sche Erkrankung (PiD), ist. Eine andere nicht-erfindungsgemäße Ausführungsform betrifft den Fall, dass die neurodegenerative Erkrankung eine Frontotemporale Degeneration (FTLD), insbesondere Ubiquitin-positive Degeneration, TDP43-positive Degeneration oder für Ubiquitin- und TDP43-negative Degenerationen, ist. Eine andere nicht-erfindungsgemäße Ausführungsform betrifft den Fall, dass die neurodegenerative Erkrankung amyotrophe Lateralsklerose (ALS) ist. Eine andere nicht-erfindungsgemäße Ausführungsform betrifft den Fall, dass die neurodegenerative Erkrankung eine spinozerebelläre Ataxie (SCA) oder spastische Paraparese (SPG) ist. Eine andere nicht-erfindungsgemäße Ausführungsform betrifft den Fall, dass die neurodegenerative/neuroimmunologische Erkrankung Multiple Sklerose (MS) oder ein MS-verwandtes Syndrom, insbesondere ADEM oder Devic-Syndrom, ist.

### Beschreibung der Abbildungen

Es zeigen
- Abb. 1a: dass die kortikale Dichte neuritischer Plaques bei ABCC1-defizienten Mäusen (ABCC1ko) um ∼75% erhöht ist;
- Abb. 1b,c: dass die mittlere Plaquegröße erhöht ist (+34%) aufgrund der größeren Anzahl an Plaques (+63%) mit einer Größe von mehr als 700µm² und einer geringeren Häufigkeit kleinerer Plaques (-24%). Fehlerbalken, Standardfehler (n≥3);
- Abb. 1d: dass die IHC-Färbung bei ABCG2-defizienten(ABCG2ko)-, ABCB1-defizienten-(ABCB1ko)-, ABCC1-defizienten (ABCC1ko)-Mäusen und bei Kontroll-Mäusen eine höhere Flächendichte an Aßbei ABCC1-defizienten Tieren zeigt. Typische Plaques derselben Größe sind im Ausschnitt dargestellt, Skalierungsbalken stellen 500µm (Übersicht) und 50µm (Ausschnitt) dar (*p<0,05);
- Abb.2a: dass die Plaquedichte im Kortex (Bedeckung) und die Größe bei spezifischen ABC-Transporter-knockout-Mäusen erhöht ist. Insbesondere ABCC1-defiziente (ABCC1ko)Mäuse zeigen eine erhöhte Aß-Amyloidbelastung (hellgraue Balken, jeweils rechts außen in den einzelnen Gruppierungen), w = Woche auf der Abszisse;
- Abb. 2b: dass die Gesamtplaquegröße bei ABCC1-defizienten (ABCC1ko) und ABCB1-defizienten (ABCB1ko)Mäusen im Alter von 25 Wochen erhöht ist, w = Woche auf der Abszisse;
- Abb. 2c: dass der Gesamtanstieg in der Plaquegröße mit dem Auftreten weniger kleiner Plaques und mehr größerer Plaques (>700 µm²) assoziiert ist, wohingegen die Anzahl mittelgroßer Plaques auf dem selben Wert bleibt, Fehlerbalken, Standardfehler (n≥5), *p<0,05;
- Abb. 3: dass die Defizienz von ABCC1 die Akkumulierung von Aβ und Aβ_{dt} fördert und dass die Aktivierung von ABCC1 (durch Gabe von Torecan) die Aß-Werte senkt; wobei
- Abb. 3a: zeigt, dass in einem Alter von 25 Wochen ABCC1-Defizienz zu einem markanten Anstieg (∼12fach) bei unlöslichem Aβ führt; und
- Abb.3b: zeigt, dass die Menge an pufferlöslichem Aβ42 in einem Alter von 25 Wochen merklich reduziert ist im Vergleich zu 22 Wochen (-56%). Dies beruht wahrscheinlich auf der Ablagerung in unlöslichen Depots. Im selben Alter ist die von Aß-Ablagerungen belegte Fläche, welche in der Immunohistochemie gemessen wird, um 83% erhöht (Fehlerbalken Standardfehler n≥5, p<0,05);
- Abb. 3c: zeigt, dass 53% der Blutgefäße schwer durch CAA beeinträchtigt sind (>75% der Gefäßwände weisen Aβ auf). Dies betrifft ABCC1-defiziente Mäuse (ABCC1 ko) im Vergleich zu 23% bei den Kontrollen (n=3);
- Abb. 3d: zeigt, dass die Expression von ABCC1 predominant im Plexus choroideus (CP) zu sehen ist, wohingegen ABCB1 hauptsächlich in den Kapillaren des Gehirns (BP) exprimiert ist;
- Abb. 3 e: zeigt, dass die Aktivierung von ABCC1 durch Thiethylperazin (Torecan) die Aβ-Werte bei Mäusen senkt (-28%), Fehlerbalken, Standardfehler (n=4, *p<0,05).

### Beispiele

### Tiere

APP-transgene Mäuse (APP, APP_{dt}) wurden von The Jackson Laboratory (Bar Harbor, USA) und der Universität Tübingen (Tübingen, Deutschland) bezogen. Die NEP-defizienten Mäuse wurden vom Riken Brain Research Institute (Saitama, Japan) bezogen. ABCG2-, ABCB1-, und ABCC1-defiziente Mäuse wurden von Taconic-Farms (Dänemark) bezogen. Alle transgenen und knockout Mauslinien wurden für mindestens 9 Generationen in den genetischen FVB-Hintergrund eingekreuzt. Die Mäuse wurden in 12h/12h Licht/Dunkelheit-Zyklus bei 23°C gehalten mit freiem Zugang zu Nahrung und Wasser.

### Methoden

### Gewebepräparation

Zur Gewebepräparation wurden die Mäuse durch zervikale Dislokation getötet und transkardial mit PBS (Phosphat-gepufferte, physiologische Kochsalzlösung) perfusioniert. Das Gehirn wurde entfernt und eine Hemisphäre in gepuffertem, 4%-igem Paraformaldehyd für Parafineinbettung und Immunohistochemie gelagert. Die andere Hemisphäre wurde in flüssigem Stickstoff schockgefroren und bei -80°C für biochemische Analysen gelagert.

### ELISA

ELISA-Kits (TH40HS, TK42HS) von The Genetics Company (TGC, Schlieren, Schweiz) wurden für die Quantifizierung von Aβ verwendet. Gehirnhemisphären wurden unter Verwendung von PreCellys24 (12 s, 6.500 rpm) homogenisiert. Nach Zusatz von Carbonatpuffer (pH 8,0) wurden die Homogenisate unter Verwendung von PreCellys gemischt (5 s, 5.000 rpm) und 90 min zentrifugiert bei 4°C und 24.000 g, um unlösliche von löslichen Aß-Spezies zu trennen. Der verbleibende Überstand (pufferlösliche Fraktion) wurde mit 8M Guanidinhydrochlorid in einem Verhältnis von 1:1,6 gemischt. Zur Extraktion der aggregierten Aß-Spezies wurde das Pellet in 8 Volumen von 5M Guanidinhydrochlorid gelöst, bei Raumtemperatur 3h geschüttelt und bei 24.000 g für 20 min bei 4°C zentrifugiert. Der verbleibende Überstand stellte die Guanidin-lösliche Fraktion dar (GuaHCl). Proteingehalte aller Proben wurden dreifach gemessen, wobei ein Nanodrop1000 Spektrophotometer verwendet wurde (ThermoFisher Scientific, Wilmington, USA). Die ELISAs wurden gemäß Herstelleranweisung unter Verwendung passender Verdünnungen durchgeführt.

### Western Blots

Für die WesternBlots wurden Gewebehomogenisate hergestellt. Die Gesamtproteinkonzentrationen der Extrakte wurden unter Verwendung eines BCA-Assays bestimmt (Pierce, Teil von Thermo Fisher Scientific, Rockford, USA). Nach der Elektrophorese von 10 µg Gesamtprotein pro Spur wurden die Proteine auf PVDF-Membranen geblottet. Nach Blockade in 5% Trockenmilch in TBST-Puffer (50 mMTris pH 7,4, 150 mMNaCl, 0,1% Tween20) für 1h bei Raumtemperatur, wurden die Blots entweder auf ABCB1 (1:500, D-11, Santa Cruz), ABCC1 (1:200, Alexis Bio) oder β-Actin (1:20.000, Sigma) über Nacht bei 4°C untersucht. Als Detektionsantikörper wurden Anti-Maus-HRP, Anti-Ratte-HRP bzw. Anti-Hase-HRP verwendet. Für die Visualisierung wurden ein Amersham ECL Plus Detektionskit und eine RoperCoolSnap HQ²-Kamera verwendet.

### Immunohistochemie (IHC)

Formalin-fixierte Gehirne wurden in Paraffin eingebettet und in 4µm-dicke Sektionen geschnitten. Nach Entfernung des Paraffins wurden die Sektionen mit einem BondMax™ Autostainer (Menarini/Leica, Deutschland) weiter behandelt. Immunofärbung wurde initiert nach Blockade endogener Peroxidase (5 min) und Epitop-Wiederfindung (epitoperetrieval) für 5 min mit 95% Ameisensäure (für Antikörper 6F3D, Dako, Deutschland) und 70% Ameisensäure (für Antikörper 4G8, Millipore, Deutschland). Primäre Antikörper wurden routinemäßig bei Raumtemperatur für 30 min mit folgenden Verdünnungen inkubiert: 6F3D (1:100), 4G8 (1:500). Primäre Antikörper wurden mit dem BondMax™ Bond Polymer Refine-Detektionskit und nach dem Standardprotokoll DAB R30 detektiert. Die Schnitte wurden vollständig digitalisiert mit einer Auflösung von 230nm unter Verwendung eines MiraxDesk/MiraxMidi-Scanners und anschließend automatisch analysiert unter Verwendung des AxioVision-Softwarepakets (Zeiss, Deutschland).

### Bewertung des Schweregrades der CAA

Gehirnschnitte von APP_{dt}-Mäusen wurden mit 4G8-Antikörper angefärbt. Zumindest zwei nicht aufeinander folgende Sektionen wurden auf CAA der Hirnhautgefäße in verblindeter Weise untersucht. Alle Hirnhautgefäße wurden manuell gezählt und der Schweregrad der CAA wurde wie folgt kategorisiert:
Kategorie I: nicht beeinträchtigt
Kategorie II: ≤25% der Peripherie positiv eingefärbt
Kategorie III: ≤50% der Peripherie positiv eingefärbt
Kategorie IV: ≤75% der Peripherie positiv eingefärbt
Kategorie V: ≤100% der Peripherie positiv eingefärbt

Die mittlere Anzahl an Gefäßen zu jeder Kategorie wurde relativ zur Gesamtzahl aufgefundener Gefäße kalkuliert.

### Endothelzellen-Transwell-Assay (ECTA)

Endothelzellen von Mausgehimkapillaren wurden wie bei Coisne *et al.* beschrieben hergestellt (Coisne, C. et al. Mouse syngenic in vitro blood-brain barrier model: a new tool to examine inflammatory events in zerebral endothelium. Laboratory Investigation; 85, 734-746 (2005)). Mindestens zehn 3-4 Wochen alte Mäuse wurden enthauptet und die Gehirne entfernt. Nach Dissektion des Hirnstamms, der weißen Substanz und der Hirnhaut wurde das Gewebe in zwei Volumen Waschpuffer B homogenisiert (WBB) (Hanks bufferedsaltsolution (HBBS), 10 mM HEPES, 0,1% BSA) unter Verwendung eines 15 ml Glassdouncers (Wheaton Industries, Millville, NJ; USA). Ein Volumen von 30% Dextranlösung wurde dem Homogenisat zugegeben. Es wurde zweimal bei 3.000 g und 4°C zentrifugiert. Das Pellet, welches die Gefäße enthielt, wurde in WBB resuspendiert und große Gefäße wurden manuell durch harsches Pipettieren der Lösung aufgebrochen. Vakuumfiltration durch 60 µm-Membranen (SEFAR, Schweiz) wurde eingesetzt um große Gefäße von den Kapillaren zu trennen. Nach kombinierter Behandlung mit Kollagenase/Dispase (HBSS, 10 mM HEPES, 0,15 µg/ml TCLK, 10 µg/ml DNAse-I, 1 mg/ml Kollagenase/Dispase (Roche) wurde Einzelzellsuspension durch weiteres harsches Pipettieren der Lösung erreicht. Endothelzellen wurden in Matrigelbeschichtete Transwellinserts eingebracht (0,4 µm Poren, Greiner Bio-One, Deutschland) mit einer Dichte von 120.000 Zellen pro Insert und wachsen gelassen auf einer unterstützenden Glialkultur.

Schwefelgelb wurde zur Bestimmung des parazellulären Flusses während der Assays verwendet. Das Kulturmedium des abluminalen Kompartments wurde ersetzt mit einer Lösung, welche 10 ng Aβ42 enthielt (1,6 nM Endkonzentration). Anschließend wurden Proben aus dem luminalen Kompartment nach 2h, 6h bzw. 24h entnommen und der Aß-Gehalt wurde mit ELISA bestimmt (TK42-highsense, TGC, Schweiz). Die Transportrate wurde wie bei Coisne *et* a/. beschrieben bestimmt (Coisne, C. et a/. Mouse syngenic in vitro blood-brain barrier model: a new tool to examine inflammatory events in cerebral endothelium. Laboratory Investigation; 85, 734-746 (2005)).

### ELISA-Statistiken

Der Lilliefors-godness-of-fit-Test (alpha=0,05) wurde auf die ELISA-Daten und auf die logtransformierten ELISA-Daten angewandt, um zwischen der Annahme von normal verteilten Probendaten und der Annahme log-normal verteilter Probendaten zu unterscheiden. Trotz der geringen Probengröße wurde für beiden Datensätze die Nullhypothese (H₀) abgelehnt für 5 von 44 Proben. In Übereinstimmung mit der Beobachtung überwiegend positiven Versatzes (skew) und strikt positiver Probendaten wurde die Annahme normal verteilter Daten verworfen. Mittelwerte und Konfidenzintervalle wurden unter der Annahme einer zugrunde liegenden log-normal Verteilung berechnet. Der Wilcoxon-Rank-sum-Test wurde angewandt, um die ELISA-Daten der verschiedenen Mausstämme für jeden Zeitpunkt zu vergleichen.

## Patentansprüche

1. 2-(R²-Thio)-10-[3-(4-R¹-piperazin-1-yl)propyl]-10*H*-phenothiazin gemäß der allgemeinen Formel I 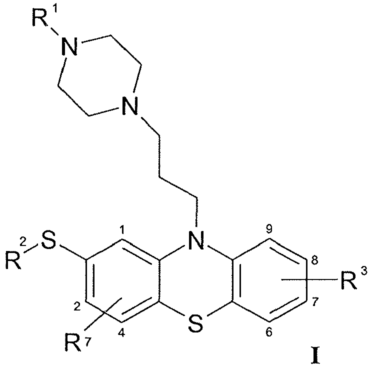 wobei
der Rest R¹ eine Methylgruppe, der Rest R² eine Ethylgruppe und die Reste R³ und R⁷ Wasserstoff sind,
zur Behandlung einer β-Amyloidopathie oder einer α-Synucleinopathie, welche mit einer zerebralen Proteinablagerung und einer verminderten Aktivität des zerebralen ABCC1-Transporters einhergehen.

2. 2-(Ethyl-thio)-10-[3-(4-methyl-piperazin-1-yl)propyl]-10*H*-phenothiazin zur Verwendung gemäß Anspruch 1, **dadurch gekennzeichnet, dass** weitere Wirkstoffe zugesetzt werden.

3. 2-(Ethyl-thio)-10-[3-(4-methyl-piperazin-1-yl)propyl]-10H-phenothiazin zur Verwendung gemäß Anspruch 2, **dadurch gekennzeichnet, dass** als weitere Wirkstoffe 1-Benzhydrylpiperazine zugesetzt werden.

4. 2-(Ethyl-thio)-10-[3-(4-methyl-piperazin-1-yl)propyl]-10*H*-phenothiazin zur Verwendung gemäß Anspruch 2 oder 3, **dadurch gekennzeichnet, dass** als 1-Benzhydrylpiperazin 1-Benzhydryl-4-cinnamyl-piperazin zugesetzt wird.

5. 2-(Ethyl-thio)-10-[3-(4-methyl-piperazin-1-yl)propyl]-10*H*-phenothiazin zur Verwendung gemäß einem der Ansprüche 1 bis 4, **dadurch gekennzeichnet, dass** die β-Amyloidopathie Alzheimer Demenz ist.

6. 2-(Ethyl-thio)-10-[3-(4-methyl-piperazin-1-yl)propyl]-10*H*-phenothiazin zur Verwendung gemäß einem der Ansprüche 1 bis 4, **dadurch gekennzeichnet, dass** die α-Synucleinopathie die Parkinson'sche Erkrankung oder Lewy-Körperchen-Demenz ist.

## Claims

1. A 2-(R²-thio)-10-[3-(4-R¹-piperazin-1-yl)propyl]-10*H*-phenothiazin according to the general formula I 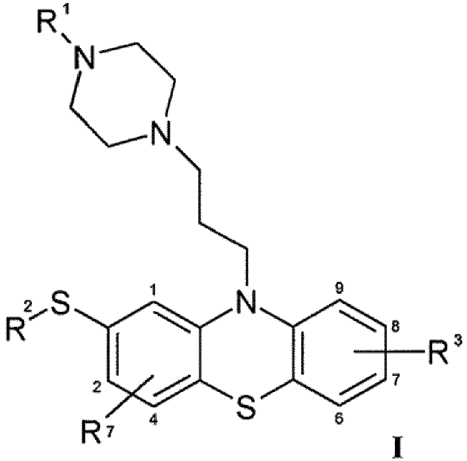 wherein the residue R¹ is a methyl group, the residue R² is an ethyl group, and the residues R³ and R⁷ are hydrogen, for treating a β-amyloidopathy or an α-synucleinopathy accompanied by a cerebral protein deposit and a reduced activity of the cerebral ABCC1-transporter.

2. The 2-(R²-thio)-10-[3-(4-R¹-piperazin-1-yl)propyl]-10*H*-phenothiazin for the use according to claim 1, **characterized in that** further active substances are added.

3. The 2-(R²-thio)-10-[3-(4-R¹-piperazin-1-yl)propyl]-10*H*-phenothiazin for the use according to claim 2, **characterized in that** 1-benzohydrylpiperazines are added as further active substances.

4. The 2-(R²-thio)-10-[3-(4-R¹-piperazin-1-yl)propyl]-10*H*-phenothiazin for the use according to claim 2 or 3, **characterized in that** 1-benzohydryl-4-cinnamyl piperazine is added as 1-benzohydrylpiperazine.

5. 2-(R²-thio)-10-[3-(4-R¹-piperazin-1-yl)propyl]-10*H*-phenothiazin for the use according to any one of claims 1 to 4, **characterized in that** the β-amyloidopathy is Alzheimer's dementia.

6. The 2-(R²-thio)-10-[3-(4-R¹-piperazin-1-yl)propyl]-10*H*-phenothiazin for the use according to any one of claims 1 to 4, **characterized in that** the α-synucleinopathy is Parkinson's disease or Lewy body dementia.

## Revendications

1. 2-(R²-Thio)-10-[3-(4-R¹-pipérazin-1-yl)propyl]-10H-phénothiazine de formule générale I 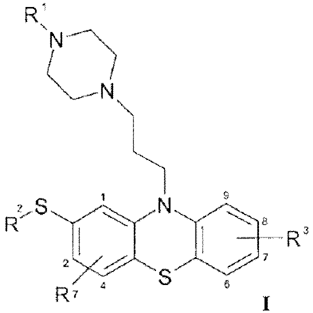 dans laquelle
le radical R¹ est un groupe méthyle, le radical R² est un groupe éthyle et les radicaux R³ et R⁷ sont l'hydrogène,
pour le traitement d'une β-amyloïdopathie ou d'une α-synucléinopathie, qui sont accompagnées d'un dépôt cérébral de protéines et d'une activité réduite du transporteur ABCC1 cérébral.

2. 2-(Ethyl-Thio)-10-[3-(4-methyl-pipérazin-1-yl)propyl]-10H-phénothiazine destinée à une utilisation selon la revendication 1, **caractérisée en ce que** des agents actifs supplémentaires sont ajoutés.

3. 2-(Ethyl-Thio)-10-[3-(4-methyl-pipérazin-1-yl)propyl]-10H-phénothiazine destinée à une utilisation selon la revendication 2, **caractérisée en ce que** des 1-benzhydrylpipérazines sont ajoutées en tant qu'agents actifs supplémentaires.

4. 2-(Ethyl-Thio)-10-[3-(4-methyl-pipérazin-1-yl)propyl]-10H-phénothiazine destinée à une utilisation selon la revendication 2 ou 3, **caractérisée en ce que** de la 1-benzhydryl-4-cinnamyl-pipérazine est ajoutée en tant que 1-benzhydrylpipérazine.

5. 2-(Ethyl-Thio)-10-[3-(4-methyl-pipérazin-1-yl)propyl]-10H-phénothiazine destinée à une utilisation selon l'une quelconque des revendications 1 à 4, **caractérisée en ce que** la β-amyloïdopathie est la maladie d'Alzheimer.

6. 2-(Ethyl-Thio)-10-[3-(4-methyl-pipérazin-1-yl)propyl]-10H-phénothiazine destinée à une utilisation selon l'une quelconque des revendications 1 à 4, **caractérisée en ce que** l'α-synucléinopathie est la maladie de Parkinson ou la démence à corps de Lewy.
